(19) Europäisches Patentamt / European Patent Office / Office européen des brevets

(11) **EP 3 626 160 A1**

(12) **EUROPEAN PATENT APPLICATION**

(43) Date of publication:
**25.03.2020 Bulletin 2020/13**

(51) Int Cl.:
**A61B 5/00** *(2006.01)*     **A61B 5/02** *(2006.01)*
**H04N 5/232** *(2006.01)*     **G01S 17/02** *(2020.01)*

(21) Application number: **18196325.7**

(22) Date of filing: **24.09.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(71) Applicant: **Dianova A/S**
**2800 Kongens Lyngby (DK)**

(72) Inventors:
• **Svendsen, Morten Bo Søndergaard**
**2800 Kongens Lyngby (DK)**

• **Hansen, Jan Bertholdt**
**2800 Kongens Lyngby (DK)**

(74) Representative: **Inspicos P/S**
**Kogle Allé 2**
**2970 Hørsholm (DK)**

Remarks:
Amended claims in accordance with Rule 137(2) EPC.

(54) **A HANDHELD IMAGING ELEMENT WITH A MOVEMENT SENSOR**

(57)     An imaging element for providing image data, such as for LSCI/LASCA measurements, the imaging element comprising: a camera, a laser, a movement detector and a processing device configured to correct the image based on the movement detected. The unit may be handheld.

Figure 1

**Description**

The present invention relates to an imaging element with a movement sensor and in particular to a handheld imaging element comprising an imaging device for generating image data and a movement sensor for movement compensation of the image data provided by the imaging device.

Imaging elements may be seen in WO2017062759, US2017017858, CN107307848, CN204765619U, WO2014009859, WO2011117779, RU2573053, US2016270672, WO2010017508, CN102357033, "Correcting for motion artifact in handheld laser speckle images", Lertsakdadet B et al., Journal of Biomedical Optics, March 2018, page 036006 (7 pp.), NR 3 VOL 23., "Development of a handheld blood flow measurement system using laser speckle flowgraphy", Min-Chul Lee et al., Optical Society of Japan, co-published with Springer, April 2015, page 308-314, NR 2, VOL 22., "Handheld, point-of-care laser speckle imaging", Farraro R et al., Journal of Biomedical Optics, September 2016, page 094001 (6 pp.), NR 9 VOL 21., and "PulseCam: High-resolution blood perfusion imaging using a camera and a pulse oximeter", Kumar Mayank et al., 16 August 2016, 38th Annual International Conference of the IEEE Engineering in Medicine and Biology Society (EMBC), page 3904-3909.

In a first aspect, the invention relates to an imaging element for providing image data, the imaging element comprising:

- an imaging device configured to output image data,

- a source of monochromatic radiation,

- a movement sensor configured to output a movement signal, and

- a processing device configured to receive the image data and the movement signal and output movement corrected image data.

In the present context, image data is data representing a physical surface or structure, such as a portion of a human or animal body, a structure or the like, and which may be represented in an at least two-dimensional fashion. In one situation, the image data may be an image of the structure or surface, such as where a colour or the actual surface/object has been replaced or added information relating to an analysis performed on the basis of the image data, such as flow or relative movement one portion of the surface/structure vis-à-vis another portion of the surface/structure.

Typically, images are 2D representations of 2D or 3D elements, where the 2D representation is a projection of the surface of the elements on to a surface or image plane. Usually, an image has, at a position often called a pixel, a colour or grey tone of a corresponding position of the element. Image data in the present context, may, at a position, comprise data or information in addition to or instead of such colour/grey tone. Such data or information may represent a value, such as a relative movement between a portion of the element at the corresponding position and other elements. As will be seen below, a vast number of measurement types may be employed, such as quantification of surface stress and the like. In a particular example, flow velocity and/or direction may be represented in the image data and in a position of the image data corresponding to a position or portion in the element where a flow or relative movement is seen vis-à-vis other portions of the element.

The image data may be represented in any manner. A large number of image formats exist, such as JPEG, TIFF, GIF, BMP or the like. In general, the image data may represent a number of portions or pixels each preferably at least representing a coordinate or position in an at least 2D surface and a value corresponding to a result of an analysis of the image data.

Preferably, the radiation source is configured to launch the monochromatic radiation on to the above 2D or 3D element, and the imaging device is able to detect radiation reflected or scattered by the element. The imaging device may be a camera, such as a video camera, which is able to detect received radiation, in a number of individual sensors, such as a sensor array. A camera may determine the received intensity and/or colour in each sensor. The imaging device may comprise a filter removing radiation with a colour or wavelength different (such as outside a wavelength interval comprising the wavelength of the monochromatic radiation) from that of the radiation source. A camera may comprise a 2D sensor, such as a CCD, or a 1D sensor which may be moved or scanned during providing of the imaging data.

In this context, monochromatic radiation is radiation having a predetermined wavelength. In this context, "monochromatic" is a situation where the radiation has a predetermined wavelength and an output intensity at that wavelength. Then, no radiation is output with an intensity exceeding 50%, such as 40%, such as 20%, such as 10% of the output intensity and with a wavelength within 10nm, such as within 20nm of the predetermined wavelength.

A source of monochromatic radiation may be a laser or a gas-discharge lamp, such as a Mercury-vapour lamp. Clearly, radiation of other wavelengths may be emitted. If the wavelength of undesired radiation is too close to the wavelength desired output, an optical filter may be employed. Additionally or alternatively, an optical filter may be positioned in front of the imaging device to remove undesired wavelengths.

Naturally, the radiation source may comprise a source and a radiation guide for transporting radiation from the source toward an emission portion from which the radiation is emitted. A radiation guide may be an optical fibre, a fibre bundle, a lens, a prism or the like.

In the present context, a movement sensor is configured to sense a movement thereof. The movement sensor preferably is attached to, secured to and/or fixed to the imaging device so that the movement sensor determines movement of the imaging device.

A movement sensor may sense any type of movement, such as translation, rotation, vibration or combinations thereof. The movement may be a movement in relation to e.g. the earth or relative to another object, such as an operator or an element on to which the monochromatic radiation is launched. The movement sensor may comprise e.g. a gyrometer, an accelerometer, or the like. Any type of measurement determining a movement may be used, such as detection of changes, relative to the sensor, of the direction of the earth's magnetic field, air pressure or the like may also be used. A vast number of very compact movement sensors exist, including sensors sensing a variety of parameters.

The movement signal may represent something as simple as a distance, an angle or a direction, but may be more complex so as to be able to describe a movement being a combination of a rotation and a translation. The movement signal may also comprise additional information, such as a direction as well as a rotation/translation. The direction, rotation etc. may be determined in relation to a fixed coordinate system, which may be that of the earth, the radiation emitter, the sensor, the imaging device or the like.

The processing device may be any type of processor, controller, DSP, ASIC or the like, as well as a combination thereof. The processing device may be monolithic or formed by a number of such elements.

The processing device is configured to receive the image data and the movement signal and output movement corrected image data.

Movement may create different types of movement artefacts in image data depending on the actual measurement set-up. If a normal camera is moved during exposure of the sensor to take a standard image, the resulting image will be blurred. The movement causes neighbouring pixels to view the same element in the imaged portrait (be that of a tree, a person, a mountain) over the time of the exposure.

In another situation, two sets of image data is obtained and the processing thereof is a comparison of the two images, such as the LSCI method. Then, movement between the deriving of the two sets of image data is also, or alternatively, desirably compensated for. Thus, the movement sensor may be configured to determine the movement between the providing of the two sets of image data. Clearly, the movement during the providing of a set or both sets of image data may also be determined. Then, the compensation may take this movement or these movements into account, as is also described further below. The movement sensing may determine how the sensor has been moved and the processing device may then calculate "backwards" to assess what each pixel would have "seen" if the camera was not moved.

In another situation, the imaging has the purpose of determining movement of portions of an imaged element relative to the imaging device. This may be used for determining relative movement of one portion vis-à-vis another portion, of the element. If no movement is detected, one portion may be stationary when another is not. However, movement of the imaging device may cause the otherwise static portion to seem like moving and cause the moving portion to seem like moving more, differently - or less.

Also, the compensation will depend on exactly what is moving. If the radiation source moves, the compensation may differ from the situation where the imaging device moves. This is described further below.

Determining the overall movement may enable the processing device to determine the artefact caused in each point or pixel and thus to remove the effect of the movement detected, leaving the relative movement in the corrected image data. Alternatively, an overall movement artefact may be determined and applied to all points/pixels in the image data.

The movement artefact created may differ for different portions of the image data. As mentioned, the image data usually represent a number of points or pixels positioned in a 2D pattern, such as a matrix.

Clearly, the movement detected may cause different artefacts in different portions of the image data depending on what the image data represent. If the image data represent a usual image with pixels and colours, translating the imaging device closer to the imaged element will not alter the data represented by the centre of the image data, as this pixel will see the same colour at all times. However, if the image data represented movement relative to the imaging device, the movement would affect also this pixel.

Thus, the movement compensation should be made with reference to what the image data represent.

A rotation of the imaging device would of course generate another artefact at the individual pixels or points. Again, this may be determined and compensated for.

The movement corrected image data may have the same format as the image data. Thus, if the image data represents a number of points or pixel values, so may the movement corrected image data. The pixel values or points may, however, be amended due to the correction for the movement detected.

Preferably, the source is configured to launch the monochromatic radiation toward a predetermined direction, the imaging device having a field of view comprising the predetermined direction at least within a predetermined distance, so that the imaging device is able to determine radiation reflected by or scattered by element(s) receiving the radiation. It is noted that the radiation emitter may be off axis with the imaging device so that it is desired that irradiated elements are at least a predetermined minimum distance from the imaging device or source.

In one embodiment, the imaging device has an imaging plane wherein the movement sensor is configured to output a movement signal correlated to movement of the imaging plane. An imaging plane often is defined by a lens or lens system imaging the scene or portrait desired imaged on to a sensor. The imaging plane thus may be defined as the sensitive surface of the sensor. However, it may be desired to provide the actual sensor remotely from the source and movement detector, such as due to size/weight/power requirements. In this situation, the imaging plane may be a surface of a fibre bundle on to which the scene is focused, which bundle may then guide the radiation to the sensor. In this situation, the movement of interest is that of the imaging plane vis-à-vis the element irradiated, as the image is subsequently "frozen" by the operation of the fibre bundle. Thus, the actual sensor may be moved relative to the imaging plane without affecting the measurement.

Size requirements may be seen in e.g. laparoscopes or endoscopes which have a narrow rod at the end of which the radiation is to be received, but where it may be desired to have the imaging device at a more remote position where more space is available.

Preferably, the imaging element has a handheld unit comprising the imaging device, or at least the imaging plane and the movement sensor. In this context, a unit may be handheld if it weighs no more than 2kg, such as no more than 1kg, such as no more than 750g, such as no more than 500g. Also, a handheld unit preferably has rather small dimensions, such as no more than 40cm along any dimension.

Additionally, or alternatively, the imaging element may be propelled such as if provided on or with a moving structure, such as a car, a vehicle, an airplane, a drone or the like.

The processing device may be in the handheld or propelled unit or remotely therefrom. Clearly, the processing device may be a central unit receiving image data and movement signals from multiple units and may then perform the correction. A handheld/propelled unit may comprise a wired or wireless connection, such as for transferring image data to the processing device, if this is not in the unit. A wired connection may be used for powering the handheld unit and/or for holding a fibre bundle, if the sensor of the imaging device is not in the unit.

Preferably the handheld unit has a handle portion configured to be grabbed by an operator, as well as a head portion comprising the radiation emitter and the imaging plane, the sensor or a lens of the imaging device.

In one embodiment, the imaging element has a triggering element configured to activate the imaging device and the movement sensor. In this manner, it may be ensured that the providing of the image data and the detection of the movement is simultaneous or at least substantially simultaneous. It is preferred that the two detections are performed simultaneously so that the movement detected is the one desirably corrected for.

Also, the trigger may be used for triggering multiple sets of image data and movement signals, as described below, image data representing different portions of an imaged element may be detected and then combined to a larger image.

In the situation with the handheld unit, the trigger may be provided in the handle part to arrive at a unit with a pistol-like shape and operation.

In one situation, the movement sensor is configured to determine a distance between the imaging element and the imaged element and wherein the processing element is configured to include the distance in the determination of the corrected image data. The distance will e.g. determine the extent of a single pixel in the image data. In the situation where the radiation emitter is configured to emit coherent radiation, the radiation will create, on the imaged element, a speckle pattern comprising a vast amount of very small high intensity spots. The distance from the imaging plane to the speckles thus will decide how much of the speckle pattern is imaged on one pixel. Also, especially if the imaging device has a fixed focus lens system between the imaging plane and the speckle pattern, it is desired that the distance is close to the focus distance in order for the image data to not be blurred. Numerous distance sensing principles are known, such as laser-based principles, time-of-travel based principles, stereo camera based and the like. Other principles would be the providing of an off-axis light source emitting a beam into the field of view of the imaging device, so that the beam has different distances to the centre of the imaged area at different distances to the imaging device.

In a preferred embodiment, the imaging element further comprises a display, the processing device being configured to control the display to display an image representing the corrected image data.

In one embodiment, the image illustrates relative movement between elements illustrated in different portions of the image.

Other types of parameters may, however be determined:

- **Mechanical stress and/or strain:** polymer processing, wind mill wings, airplane wings, polymer tubing, water pipe and similar tubing connection (construction and HVAC)
- **non-uniform coating:** paint that has not yet dried, coating process failed, ointment application, degradation of coating (e.g. optics), print (2D + 3D)
- **presence and characterization of biofilm:** (bacteria, fungus or microalgae): dentistry, chemical processing plants, subjects submerged in water (boats, etc.), bioreactors, hygienic (proper cleaning of instruments or devices, need for cleaning), camera lenses

In many situations, the surface of an element may represent also parameter of the deeper lying structure, such as stress in a surface of a metallic or polymer tube may be the indication of fractures or the like in the tube material.

Additional parameters and analysis methods are described below, naturally, the display may be fixed to the movement sensor, such as if provided in the above handheld unit.

Also, providing the display in the handheld unit will allow the operator to focus on the job at hand without needing to revert his/her attention to a display positioned away from the operator or the imaged element.

As mentioned above, the image may represent different movements, such as different velocities, in different manners. In one manner, different colours indicate different velocities. Often, red is used for more extreme cases, such as higher velocities, where yellow, then green and then blue often represent lower and lower values. Alternatively, arrows may be used, the length thereof may represent the velocity or the movement. The direction of the arrow may then indicate the direction of the movement.

Naturally, the velocity indicated may be relative to any coordinate system or element. In LSCI, the velocity is relative to the camera or image plane. However, as many portions of the element imaged will be stationary relative to the camera or image plane, the relative velocity will also be relative to such portions. Velocity may be simply indicated (present or not - such as compared to a threshold velocity) or may be quantified.

Monochromatic radiation may be used for a number of different types of analysis, such as where an absorption of the radiation from the source is determined or where the radiation from the source causes fluorescence which is then determined.

Methods based on such techniques may be used for a variety of analysis types.

In one situation, the diameter of a deep underlying structure is determined in a multi-layered object, where the layers closest to the sensor are fully or partially transparent to the monochromatic light. In medicine, this is done using radiation from the infrared spectrum for detecting and measuring blood vessel structure. As a more general term, this is called spectroscopy imaging. Motion artefacts will in this case cause blurring and thus affect the resultant measurement of the underlying structure.

Further, in applications including determination of fluorescent structures, motion artefacts presents the same errors as when monochromatic light is used for imaging different absorption. The difference being, that different substances presents different (auto)fluorescence enabling detecting of bodies of different properties than the surrounding material or bodies.

Detecting and localisation of sand and stones in corn, and determining if the sand is above or below a threshold requiring removal may be obtained using e.g. (auto)fluorescence. Sorting out different minerals in a sample, where measurement enables immediate quality control and thus pricing and prioritisation option. Detecting, identifying, and/or grading, such as using fluorescence, cells in a multicellular organism. Finding and evaluating parasites in host organisms (parasites and hosts organisms comprising any combinations of assemblies of virus, assemblies of eukaryote cells and/or assemblies of prokaryote cells in a matrix or array of virus, eukaryote or prokaryote cells).

All of the above in the connection of measuring the foreign body (auto)flourescent intensity and physical size - in relation to detecting, localising, identifying and making a decision for removal or not. However, movement will cause blurring which may be compensated for in order to arrive at a sharper image.

Additionally, the movement may cause a variation of the intensity of the radiation from the source in different portions of the element irradiated. This again will cause a variation in the absorption and fluorescence and thereby in the intensity received by the imaging device in each point or pixel. Thus, the movement may in addition to blurring give rise to a compensation across the image data of the intensities represented thereby.

As mentioned above, a preferred radiation source is a laser, as it emits monochromatic radiation.

The radiation from a laser actually is coherent, which brings about even further advantages. Launching laser light or coherent radiation on to an element automatically will generate a speckle pattern on that element. Speckles are formed by the positive and negative interference of the coherent radiation on the element, as is described further below.

Using a coherent radiation source thus opens for a number of interesting analysis types bases on this speckle pattern. These are described below.

A number of further embodiments are of interest. For example, it may be desired that the processing device is configured to, if the determined movement does not exceed a first threshold, output, as the corrected image data, the first image data. Then, no correction is made, if the movement sensed is too small. Also, it may be desired that the processing device is configured to, if the determined movement exceeds a second threshold, exceeding the first threshold, not output corrected image data. Movements may be so violent that it is not possible to perform a suitable correction. Thus, the original image data may be output, so that the operator can verify this.

A second aspect of the invention relates to a method of obtaining image data of an element, the method comprising:

- launching monochromatic radiation on to the element,
- obtaining first image data of the element, using an imaging device, while determining movement of the imaging device,
- based on the determined movement and the first image data, generating movement corrected image data.

In many situations, the element is a part of a person or animal, such as an exposed part of the person or animal. The exposed part may be a wound, such as during operation. Alternatively, the exposed part may be of a person's or animals skin. Other types of surfaces of interest may be liquid surfaces or the like. Above, a vast number of surfaces and elements are described in relation to which parameters may be derived.

As mentioned above, launching monochromatic radiation on to the element facilitates the use of a number of measurement or analysis methods.

However, movement between the camera or image plane and the element may deteriorate the quality of the analysis results, so that movement is detected during the providing of the image data. Preferably, the movement is detected simultaneously with the providing of the image data.

The detected movement preferably is relative to particular portions of the element or relative to the camera or image plane. If portions of the element are stationary relative to the camera/image plane, this would be the same thing.

Obtaining image data may be performed by using the imaging device described above.

Determining the movement may be as described above. The determined movement may be indicated in the movement signal described.

Generation of the movement corrected image data may also be as described above.

In one embodiment, the imaging device has an imaging plane and wherein the step of determining movement comprises determining movement of the imaging plane vis-à-vis the imaged element. This is described further above.

As described above, the movement may be determined during obtaining of image data and/or between the providing of two sets of image data. When multiple sets of image data is provided, the generation of the corrected image data may take into account the movement between the two sets of image data. Clearly, also the movement during the providing of the (one or both) sets of image data may be taken into account.

In one embodiment, the movement determination comprises determining a distance from the imaging device, such as the imaging plane, to the imaged element, the generating step comprising generating the corrected image data also based on the distance determined. This distance is relevant in certain measurement types and especially when the providing of the image data is performed using a fixed focus.

In one embodiment, the method further comprises the step of displaying an image representing the corrected data. In one embodiment, the image data represent relative movement between different portions of the imaged element. This may be as described above. The image may illustrate movement in a number of manners. Also, movement may be quantified, such as using colours, arrows or the like.

In the methods relying on difference in absorption or difference in fluorescence the motion correction assists in correction of perceived size and peak intensity/absorbance. Motion artefacts during recording results in blurring that will make the objects of interest appear disformed as compared to their natural shape. Similar artefacts will result in decreased measures, and wrong location of peak intensity/absorbance. If the motion artefact is within a set range of motion, the correction can be applied to the calculated size based on prior knowledge on the imaging characteristics (angle of view) and distance to object.

In one embodiment, the launching step comprises illuminating the element with coherent radiation. This radiation often stems from a laser.

Using coherent radiation facilitates the use of a number of laser speckle analysis methods as described further below, as coherent radiation will create a speckle pattern on the irradiated element.

As mentioned above, it may be desired, if the determined movement does not exceed a first threshold, to output, as the corrected image data, the first image data. Thus, when the movement is too small, no correction is made. Also, if the determined movement exceeds a second threshold, exceeding the first threshold, it may be desired to not output corrected image data. This may be due to the movement being so violent that no correction is possible.

Another aspect of the invention relates to an imaging element for providing image data, the imaging element comprising:

- an imaging device configured to output image data,

- a source of coherent radiation, and

- a processing device configured to:

  - receive the image data,

  - determine, based on the image data, a movement, and

  - based on the determined movement, generating movement corrected image data.

Naturally, all aspects and embodiments may be combined.

In this context, the imaging device, the processing device, and the source may be as those described above.

Coherent radiation has the advantage that it will produce a speckle pattern on the irradiated element. Speckles are high intensity radiation spots created by positive interference. These spots are visible in the image data.

In this aspect, the movement is determined from the original image data. This may be performed in a number of manners.

Clearly, movement will be visible as a blurring of such spots. Actually, movement during the exposure time will cause the spot to instead become a line or streak, the length of which is indicative of the movement and the direction of which is indicative of the direction of movement.

Then, different portions of the image may have streaks with different lengths, as different portions may have moved differently during exposure of the image.

Naturally, a rotation and a translation will give different streaking patterns.

However, based on the movement determined, the image data may be corrected. This may remove the streaks but may also facilitate deriving other information from the image.

Speckle patterns are used for a number of different analysis, as is described below, such as LSCI/LASCA. Being able to perform movement analysis, as is described both below and above, enables the use of a handheld unit instead of the stationary, legacy units.

Thus, this manner of determining the movement is an alternative to that described above, but the results and the operation may be as that described above, as the movement correction again gives the advantages mentioned.

Below, the different types of movement are described.

A final aspect relates to a method of obtaining image data of an element, the method comprising:

- launching coherent radiation on to the element,
- obtaining first image data of the element, using an imaging device,
- based on the first image data, determining a movement and, on the basis of also the determined movement, generating movement corrected image data.

In this context, the launching step and the step of obtaining the image date may be as described above. Now, however, not separate movement determination is made. Instead, the movement is determined from the first image data.

Then, the first image data may be corrected based on the movement determined and corrected image data output.

Clearly all the above and below advantages and steps may be used also in relation to this aspect, as merely the movement determination is made in a different manner.

The movement determined is as that described above and below.

In the following, preferred embodiments will be described with reference to the drawing wherein:

- Figure 1 illustrates an area 30 illuminated and imaged by a LSCI sensing head
- Figure 2 illustrates a portable sensing head.

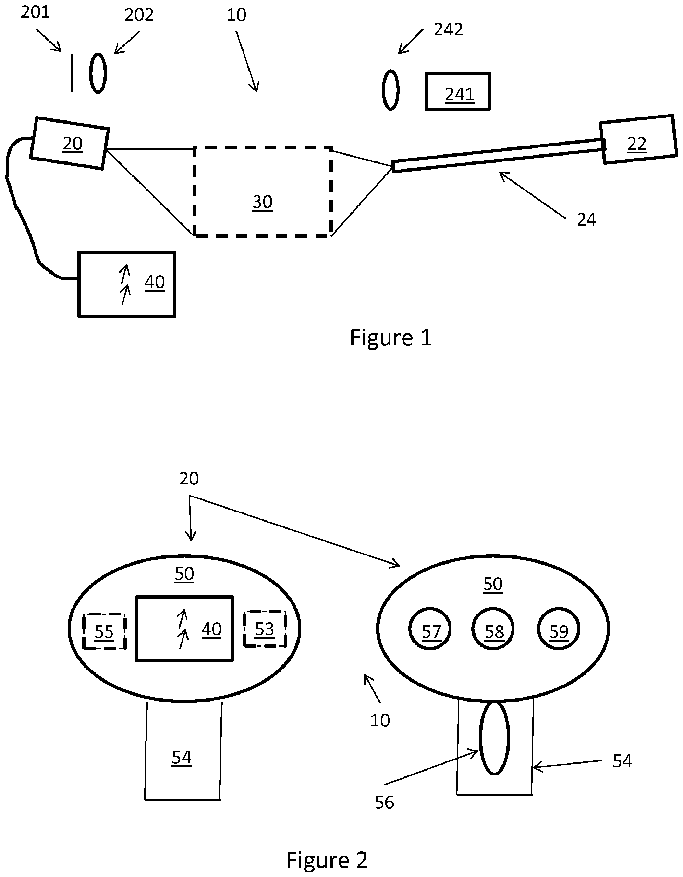

In figure 1, a Laser Speckle Contrast Imaging (LSCI) or Laser Speckle Contrast Analysis (LASCA) system 10 is illustrated comprising a sensing head 20 launching a beam forming a speckle pattern on an element 30, such as a surface portion of a human or animal body. LSCI is used for e.g. illustrating blood flow in tissue, such as during operations or wounds.

As mentioned above, a large number of other applications exist, such as so-called bio-speckle which has also been used for estimation of fruit quality, biofilm characterization, polymer stress evaluation, dental health assessment (plaque), assessment of paint drying, assessment of crystallinity, surface roughness estimation.

Perfusion measurement in medicine is used for e.g. determining regional microvascular blood flow in different bodily tissues. Its usage ranges from assessing severity of burn wounds to monitoring ulcers on diabetic patients.

Perfusion assessment and quantification of microvascular blood flow has among others been based on optical coherence tomography, laser Doppler velocimetry, and laser speckle imaging.

Laser Speckle imaging is a well-established technology, where a coherent light source emits a beam forming a speckle pattern arising from the physical nature of coherent light. This pattern is recorded via a camera, and then several different algorithms can be used to analyse the recorded speckle pattern to determine spatio-temporal changes in the element on which the speckle pattern is provided. Depending on the algorithm used, the changes of the spatio-temporal patterns, caused by blood flow, can be more or less correlated to the absolute level of blood flowing in the tissues. These algorithms all relate to the laser contrast. Common names for similar technical implementations are Laser Speckle Contrast Analysis (LASCA), Laser Speckle Contrast Imaging (LSCI), Speckle Flow Index (SFI) and in non-medical terms, the same implementations of using laser speckle to quantify biological phenomena is called Bio-speckle.

Common to the above technologies is that they relate to interferometry, causing motion artifacts if relative motion occurs between the measurement device and the tissue measured on. These motion artifacts will increase the error in the measurements of the perfusion assessment.

To avoid this, legacy laser speckle devices are bulky, mounted on sturdy arms, and possess a range of settings intended for changing the recording settings for the most optimal for a given clinical situation. However, all this is severely restricting in relation to the use of laser speckle analysis in e.g. hospitals, as the time consumed in positioning the equipment, applying settings, recording and then making a decision based on the measurement is simply too troublesome for the technology to be used in situations other than monitoring cases - such as the aforementioned burn wounds and diabetic feet ulcers etc, etc. Also, often, the analysis is desired from different angles.

The present sensor head 20 is portable, such as hand held, making it much faster and versatile in use. Also, viewing from different angles etc. is facilitated. However, relative movement now is a larger problem.

The sensor head therefore has a movement sensor for outputting information relative to movement of the sensor head. This movement may then be compensated for in the analysis.

In figure 1, to the left side, a sensor head 20 is illustrated launching radiation on to an area 30 and imaging at least that area while logging movement of the sensor head relative to the area 30. A processor calculates the resulting image which is illustrated on a monitor 40. In this image, relative movement of some portions of the area 30 will be seen relative to other portions thereof. In the image, the central area has an upward flow (arrows) compared to outer portions (no arrows).

The movement sensed may be that of the sensor head 20 relative to the earth, assuming that the area 30 is stationary relative to that coordinate system. Actually, the relevant portion of the sensor head may be a lens or image plane of the sensor in the sensor head. Above the sensor head, an image plane 201, such as the actual image sensor or CCD, is illustrated together with a lens or lens system/assembly 202 imaging the area 30 on to the image plane.

To the right in figure 1, another system type is seen also launching radiation on to the area 30 but having a remotely positioned camera or radiation sensor, now provided in a portion 22. Then, an optical fibre bundle 24 is used for guiding radiation from the left portion to the camera. In front of the left portion of the fibre bundle 24 is a lens 242 again imaging the area 30 on to the left end 241 of the fibre bundle. Then, the image plane is that defined by the lens 242 now on the end of the fibre bundle. In this manner, the portion 22 may be moved in any manner in relation to the area 30 without affecting the imaging thereof. Set-ups of this type may be used in laparoscopes, rigid endoscopes as well as in the tips of flexible endoscopes. Clearly, in this type of set-up, the coherent radiation may also be fed in fibres from the portion 22 to the end 241 for launching on to the area 30.

As mentioned above, the present set-up may be used for a wide variety of analysis and used in connection with a large number of processes. For example, the present set-up may be provided on a propelled vehicle such as a a remotely operated vehicle, such as a drone. The movement may therefore be the movement of the vehicle.

In figure 2, a sensor head 20 is illustrated having a head portion 50 and a handle portion 54. The back side of the head portion has a display 40. At the front side, a source 57 of coherent radiation is provided together with a camera 58 and a distance sensor 59. A trigger 56 is provided for the operator to activate the imaging. The overall shape is pistol like making handling and activation as well as viewing of the display simple.

A movement sensor 53 is provided in the sensor head. Also, a processor 55 is provided for receiving the output from the movement sensor, the camera, the trigger, and the distance sensor and for controlling the source 57 and generating the movement corrected image data fed to the display 40.

Clearly, the distance sensor is not required, but the quality or contrast of the speckle pattern provided by the emitter 57 will depend on the distance between the emitter 57 and the area 30. Then, it may be desired to provide a feedback, such as via the display 40 or via e.g. another visible indication (LED) or a sound for an operator to indicate when the distance to the area 30 is as recommended or e.g. is too short or too large. Also, clearly, the distance may be used in the compensation of the image data, as a rotation of the head 50 will generate different movement artefacts for different distances to the area 30.

The distance sensor may be implemented in a number of manners. Naturally, optically based distance sensors already exist, such as based on time-of-flight measurement of radiation/sound emitted by the sensor, reflected by the object/element 30 and detected by the sensor. A number of other manners exist, however, such as triangulation-based methods or by simply providing a radiation emitter off-axis from the camera and with a beam having an angle to an axis of the camera. The beam centre thus will, at different distances, be at different positions vis-à-vis the centre of the image. Also, in the LSCI situation, the speckle pattern provided on the element 30 will depend on the distance between the radiation source and the element 30 - and may then be determined from the image created by the camera. Thus, no separate distance sensor is then required.

The radiation source 57, for LSCI analysis for example, provides a speckle pattern on the area 30. A speckle pattern is generated by coherent radiation due to positive and negative interference. This coherent radiation may be fed to the area 30 directly from a radiation source, such as a laser diode, or guided to the head 50 or area 30 by an optical fibre. Naturally, the radiation may, before impact on the area 30, be reflected on a stationary or moving surface, such as when scanning the radiation on the area 30.

The movement sensor 53 may be an accelerometer, gyrometer, rotation sensor, IMU (Inertial Measurement Unit, AHRS (Attitude and Heading Reference System) or the like, such as solid-state or MEMS based sensors. Preferably, 3D movement/translation/rotation is sensed. As mentioned, a distance sensor may be implemented by the movement sensor 53 or separately therefrom. Movement compensation will depend on the movement in question as well as the parameter(s) represented by the image data. If the image data is a usual image, as described above, blurring may be caused as well as an intensity variation in the individual pixels. On the other hand, if the determination is speckle based or based on coherent radiation, the movement may cause a difference in the speckle pattern and thus the homogeneity and contrast thereof.

In the following, different types of movements will be described with reference to the type thereof in relation to a coordinate system having the Z axis from the image plane to the elements irradiated and with the X and Y axes in the image plane. Then, the effect of the movement on the image data may be determined. From this, the compensation will be clear.

*If the movement is translational along the Z axis.*

If the translational motion is purely in the Z axis, it corresponds to a change in distance to the object (d), and the deformation caused by the artefact directly related to the angle of view defined by the distance and focal length(f)

$$\alpha = 2 \cdot \arctan(\frac{d}{2 \cdot f})$$

Where and approximation to the size (Si) can be made knowing the focal length, apparent size (the wrong one) (S0) and change in distance to the camera.

$$S_i = \frac{S_0 \cdot f}{\Delta d}$$

For e.g. absorption applications, the intensity (I) change of reflected light reflecting, or light emitted from the object, is corrected (Ic) using the inverse-square-law and the distance change

$$I_c = \frac{I}{\Delta d^2}$$

For (auto)fluorescence applications, the intensity of the emitted light reaching the object, determines amount of (auto)fluorescence, thus if this is the application the recorded intensity needs to be corrected twice - resulting in the cubed distance

$$I_c = \frac{I}{\Delta d^4}$$

*If the movement is translational in the XY plane.*

If the translational motion is purely in the XY plane, parallel to the imaging plane, there is no change in size and artefacts will depend on the degree of movement artefact and the exposure time of the imaging device. Thus compared to a image taken without movement, the determined size of the object can be corrected to the expected size, without movement using only the exposure time (t) and recorded motion (v) to provide the apparent distance travelled during the exposure (L) and the determined centerline width (w) of the imaged object.

$$A_c = \frac{A_d}{w \cdot L} = \frac{A_d}{w \cdot v \cdot t}$$

*If the movement is rotational around the Z axis*

If the movement artefact arises from motion artefacts solely based on rotation around the Z-axis. Then the placement of the object in relation to the optical axis determines the degree of correction. If objects are at the rim of the image, a larger apparent movement is observed, and if the object is placed perfectly in the center of optical axis then no correction is needed unless the object is elongated. The rotational artefact will appear as streaks as in the XY-translation artefact, however when sensor readings determine rotational movement, the streaks may be considered as arc lengths (s) instead of linear displacement. Appropriate points on the edge of the object can be transformed from arc length to displacement ($\theta$), when knowing the location of the object in relation to the optical axis (r).

$$\theta = \frac{s}{r}$$

When the arc length has been converted this way to displacement, then the same approach as described above in translational motion can be utilized.

*If the movement is rotational around the an axis in the XY plane*

If the rotational motion is in the XY plane, the deformation caused by the artefact is directly related to the angle of view defined by the distance and focal length(f)

$$\alpha = 2 \cdot \arctan(\frac{d}{2 \cdot f})$$

The motion artefact causes apparent motion and deformation as if the object is moving on a sphere determined by the optical angle and distance to object. This will result in the same issues and solutions as above, but also a change in perspective.

If the emitted light is covering the imaging field in a non-uniform but known fashion (partly illuminated, circle, square, difference in intensity), then the registration of the difference in expected light field and the observed radiation can be used to determine if there is an angulation and thus observed perspective. This change in perspective on size can be corrected using a programmatical implementation of orthogonal projection correction. Then the intensity of the light from the object can be corrected in the perspective gradient using the intensity equations above.

In addition to the above movement which usually will be between the imaging device and the irradiated element and/or the earth, movement may also take place between the radiation source and the irradiated element. This may give rise to a variation in the radiation intensity over the irradiated element. From the movement and e.g. the emission intensity characteristic (often Gaussian for lasers), the intensity variation over the irradiated element and thus the image data may be determined. This, naturally, may cause a variation in the intensity of radiation received over the image data, such as when this radiation is reflected/scattered by the irradiated element or when the radiation emitted causes e.g. fluorescence to be emitted from the irradiated object.

Usually, the movement will be a combination of the above movement types. However, this movement may be de-composed into the above types, where after compensation may be performed.

This type of artifact may be added to the others and a combined compensation performed.

When the radiation is coherent so as to e.g. generate a speckle pattern on the irradiated element, the movement may cause other or additional artifacts. The speckle pattern is generated by the interference of the coherent radiation with itself on the irradiated object. Thus, movement of the radiation source relative to the irradiated object will cause a variation in the speckle pattern. Movement of the imaging device may be handled as above.

*Movement of the coherent radiation source relative to the irradiated object*

Movement of the radiation source will create a variation in the speckle pattern. A distance variation of half the wavelength will give dramatic variations in the speckle pattern, so compensation is desired.

When e.g. determining relative movement, the speckle pattern is imaged. Movement will be a movement relative to the radiation source and will thus vary the speckle pattern, whereas stationary portions will retain the speckle pattern. A simple manner of quantifying the movement would be to determine the contrast in different portions of the image. A high contrast is seen where no change is seen in the speckle pattern. A low contrast will be seen where the speckle pattern varies a lot - i.e. where the movement is large.

As this is very sensitive to variation, a very low exposure time is often desired.

For each portion of the irradiated element/object, such as for each portion or pixel in the image data, the distance variation between the radiation source and the portion of the irradiated element/object may be determined. This will describe the variation created in the speckle pattern at that particular portion, as the speckle pattern is generated by positive/negative interference of the radiation (and thus depends on the wavelength). This again may describe a contrast or homogeneity in that particular pixel or portion. This will be taken as a sign of movement (if the parameter sought for is movement) in addition to the movement of the portion itself (the parameter desired).

If motion occurs while the providing of image data, it will impact algorithms using spatial contrast analysis, such as LASCA. If movement occurs, then translational artefacts will be a linear function of recorded motion depending on imaging and light source configurations and the correction needs to take place using motion dependent device specific calibration ($\varepsilon_b(m)$)

$$ K = \varepsilon_b(m) \frac{\sigma_I}{\mu_I} $$

Where $\delta/\mu$ is the standard manner of determining contrast (standard deviation divided by mean value) and $\varepsilon$ is the sum of the errors. K then is the resulting value, such as a velocity.

*Motion between sets of image data*

Analysis types, such as LSCI exist where image data is taken twice, and the image data compared. This is an alternative to situations where portions of the same image data, such as in the LASCA technique, is "internally" compared.

Using two sets of image data, differences from one to the other may be caused by movement and may thus be used for quantifying this movement.

Movement of the measuring set-up however then will create an artifact.

In between image motion causes severe artefacts for time contrast algorithms such as LSCI caused by a shift in object location in the image being perceived as a large regional motion. Transforming images using the abovementioned camera correction, will allow regions of objects to be transformed into a corrected shape and location minimizing this error. One can also average over several time contrast images to obtain a smaller but similar effect.

Rotational artefacts creates a change in angle. Translational motion between frames is not an issue for the light source, more than it is an issue of object location in the image.

The sum of motion artefacts is based on object motion, source motion, and imaging device motion. Knowing the motion of imaging plane and light source, will enable easier correction for object motion artefacts, as a function of source separation of the sources for errors.

Having then corrected for the movement, the analysis of the image data may be performed.

Depending on the camera setup, the recorded image might need to be split from an image with multiple channels or color-intensity representation (e.g. RGB, CMYK, HSV, etc) into a single channel grey scale image where the grey scale represents the monochromatic light intensity.

When the image has been converted, in general there are two ways of calculating laser contrast. The contrast calculated in each pixel over time, i.e. in between different images, this time varying speckle is often denoted LSCI (Laser Speckle Contrast Imaging) or LSI (Laser speckle imaging). Within one image the laser contrast can be calculated by dividing the image into smaller regions e.g. 5x5 pixels, and calculating the contrast within these. This spatial contrast is often denoted LASCA (Laser Speckle Contrast Analysis). There also exists combinations of the two implementations for calculating laser speckle contrast, such as Laser speckle temporal contrast analysis (LSTCA), Laser speckle flowgraphy (LSFG), Laser speckle perfusion imaging (LSPI), temporal LASCA (t-LASCA) and modified LSI (mLSI), and simplified implementations like Speckle Flow Index (SFI).

A preferred manner of performing the actual calculations of the perfusion data or relative movement data is described below:

The degree of blurring of the speckle pattern is quantified by the speckle contrast. When exposure time of the camera is longer than the time scale of the speckle fluctuations recorded image results in a blurring of the speckle pattern in areas of high motion. Using a Laser Speckle Imaging as perfusion measurement system an algorithm determining the laser contrast may be defined as:

$$K = \frac{\sigma_I}{\mu_I}$$

If instead examining the changes in the speckle pattern over time, and depending on the theoretical flow distribution, the contrast equation can take different forms; relying on Gaussian, Lorentzian

$$K^2 = \beta \left[ \frac{t_c}{T} + \frac{t_c^2}{2T^2} \left( \exp\left( -\frac{2T}{t_c} \right) - 1 \right) \right]$$

In regions with a given pixel size, $\mu$, is the average intensity of each region and sigma is the standard deviation of pixel intensities in each region. The flow index, such as the Speckle Flow Index, is then calculated for each region to transform the contrast values in to statistics that match blood flow

$$SFI = \frac{1}{T \cdot K^2}$$

Where T is the exposure time and K the above contrast.

Regional difference in the laser pattern, can also be described using statistics arising from a co-occurence matrix (constrast, dissimilarity, homogeneity, angular second moment, correlation and energy). This has been demonstrated using the kurtosis value (C) in image regions

$$C = \frac{\mu^4}{\sigma^4}$$

The kurtosis is a different measure that describes the shape of a distribution based on the ratio between the central tendency and the deviation of the data, and as such related to the inverse of the contrast (K). Its advantage is that imaging will have a higher signal-to-noise ratio, appearing less noisy, and providing a more precise foundation for segmentation of underlying objects.

There are also methods for applying a general correction to the contrast images. This is more simple implementations than the specific motion correction types as described above. However they are used best when wanting to measure the contrast, or change in contrast within a region-of-interest, or in an image where the object is quite uniform in structure (e.g. a surface containing drying paint).

Data from the motion sensor (rotational via the gyroscope in the IMU and translation via the accelerometer) is processed, obtaining a Vectorial motion (rotation for gyroscope) statistic (Ve) summarizing the XYZ motion utilizing the Pythagorean distance

$$Ve_x = \sqrt{X_x^2 + Y_x^2 + Z_x^2}$$

Utilizing rotational and translation measures from the motion sensor to directly correct values over a time series and/or in two dimensional images and a combination hereof. Correction for small translations, or rotations, of the image contrasts can be done using the below equation

$$K_{corrected} = \frac{K}{c \cdot Ve_x}$$

Where C is an equipment specific calibration value.

This implementation will allow to correct single ROI values for motion artefacts, or be applied over the whole image if motion is below a threshold where the image still contains the same objects.

Likewise, for time series, or time-based contrast analysis, frequency distributions from the motion data can be extracted using e.g. fast-Fourier transformation. The frequency distribution can then be used to automatically select the frequency representing the most powerful signal, and then using digital filtering like a Butterworth filter to remove the motion frequencies from the contrast data series.

Active noise reduction

Even though motion of the coherent light source represents motion artefacts, intentional changes to the coherent light source can be made for the purpose of obtaining a higher resolution image. The visualisation of the laser speckle contrast can be further improved by using active noise reduction, by modifying the coherent light source or what it is reflected on towards the object of interest. The active noise reduction both has an advantage in situations without device motion and in situations including device motion. In situations without motion, the controlled changes of the light emitted functions as a running average when using time based contrast analysis.

The whole device could be motion dampened by mounting it on a mechanical gyro, however both the camera and the light source could be mounted on separate gyros or similar mechanical contraptions allowing for active control of the device parts based on the motion of the device mount as each element needs specific correction approaches depending on the nature of the motion.

Likewise with it is possible for modifying projected light based on the motion, either changing the direction of the projector, or moving a diffuser in front of the light source according to the motion of the whole device.

The camera may have a fixed focus length in order to make the operation very simple. In this manner, the distance sensing may be used for guiding the operator to provide a correct distance, where after the camera will be "point and shoot". This feedback may be audible or visible such as on a display, on separate visible indicators (red/green LED for example). In fact, in all embodiments radiation may be launched on to the area 30 and thus visible to the operator, indicating whether the distance is OK or not. For example, if the distance is not OK, a red dot, cross or the like may be visible on the area 30 - and when it is no longer there, the distance may be OK.

The trigger has multiple advantages, such as to allow the operator to obtain the data when desired. Also, the coordination of the imaging and the movement sensing (or the logging or correlation of such data) may be handled by the trigger.

A feedback may also be given to the operator when pressing the trigger as to whether the image taken is acceptable or not. For example, if the distance was inacceptable, if the movement was too large, if the laser speckle contrast was not sufficient, or if any other parameter was not within range, an "unsuccessful" sound could be output (or a corresponding other indication could be made), so that the operator need not refer to a display to know that another "shot" is required.

Clearly, if the movement sensed was too large for a good compensation to be made, it may be desired to simply output the image data without movement compensation - or to discard the data altogether.

Also, if the movement sensed was below a threshold, no movement compensation need be performed, so that the usual perfusion image is provided.

Multiple images may be provided between which or during which movement takes place. This movement may then be used for determining the relative positions of the portions of the element 30 imaged in different images, which may then be combined or stitched into a single image if desired. Thus, zoomed-in or higher resolution images may be assembled to a larger image.

Other types of analysis or measurements may be added to the present system. Absorption of radiation by the area 30 or elements therein, such as blood or oxygen, may be used for determining e.g. the presence or concentration/amount of e.g. molecules therein. Radiation may be emitted from the head 50 and detected by the camera, where absorption of the radiation may indicate the concentration of oxygen (oxygenation) for example.

Also, visible radiation may be used for indicating demarcations on the element 30 so that the operator may see the boundaries of the image or camera viewing angle, demarcations of the boundaries of the speckle pattern, indications of whether the distance should be increased/decreased, whether other analysis, such as the oxygenation analysis, is turned on or off. Then, the operator need only point the head portion toward the area 30 but need not view the display 40 or other indications on the handle in order to know the mode of operation, the pointing direction as well as the acceptability of the measurement/analysis which may then be activated using the trigger.

Actually, multiple speckle patterns may be provided, such as sequentially, by launching multiple radiation wavelengths on to the area 30. Providing multiple flow measurements may increase the overall precision of the analysis. The same camera may be used (as may the same source of coherent radiation if it has multiple wavelengths), as the wavelengths of the two speckle patterns may be separated due to their different wavelengths.

Separation can be fulfilled using optical filters, matching the emitted light (bandpass, lowpass, highpass or an appropriate combination), placed in front of a camera that is sensitive to a wide range of wavelengths. This implementation enables that the filters can be changed to the appropriate wavelength for a given situation (light condition, measured quantity, surface characteristic, relative motion regime, distance to subject, or in the presence of fluorescent materials in the illumination area). With several light sources this can be done by multiple cameras each equipped with a specific optical filter, or by a mechanical implementation placing the filters in the imaging plane.

Multiplex controlling light sources and coordinated recording with camera may be performed, such that the camera records and handles separate light sources independently. This would provide the same benefits as with optical filters.

Using camera setups with multiple chips is also possible, where the light sources are separated using a prism construction in connection to the imaging plane. The prism will cause the different wavelengths to arrive at the difference sensors in the camera, effectively separating the emitted light sources. This again will provide the same benefits as with optical filters.

Yet another method is Laser Doppler Flowmetry (LDF) which essentially measures the same as Laser Speckle imaging, the primary difference being which side of the contrast equation is measured, thus implementations using LDF combined with a motion sensor can equally be corrected for by the abovementioned approach.

The optional distance measurement may be performed using Laser Triangulation. This would also enable high precision movement measurements, as the operator motion can be calculated and subtracted. In a different embodiment, the movement of the head 20/50 relative to the element 30 is determined solely from the image data provided by the camera. As described above, the speckle pattern on the element 30 is defined by the coherence of the radiation and thus forms a pattern of spots created by negative and positive interference of the radiation when scattered/reflected. This pattern clearly will vary on the element 30 depending not only on the distance but also on the angle between the head (the radiation source) and the element 30. Thus, this pattern will vary, if the head or radiation source is moved relative to the element 30. This movement thus may be determined based only on the variation of the speckle pattern.

Within an image the motion can be assessed as the exposure time, and distance to the subject is known. If relevant motion occurs within the exposure time, a speckle spot would be visible in multiple pixels; streaky blurring occurs, and the length of the streaks can be translated into to motion by

$$v = L \cdot t$$

Where L is the streak length, and t the exposure time, providing the velocity v, where the streak direction represents the direction of relative motion. Depending on the specification of the camera, programmatical correction is needed if the sensor is of a progressive scan nature i.e. not exposing all parts of the sensor at the same time.

From the movement determined in different portions of the image, also rotation of the camera vis-à-vis the illuminated element may be determined.

To assess the relative motion between two or more images using only optical means, it is generally needed to identify and match specific regions of each image and then calculate the distance each identifiable region has moved. Knowing the time between each image is then used to calculate the velocity and or acceleration of the movement. To assess the relative motion between images, methods utilizing optical flow can be implemented.

To identify different regions of an image, which matches regions in another image, an optimization approach is normally used. The optimization can be completed in fixed size blocks, or in areas of varying size, the comparison of regions can then be made by calculating the difference in regions. A match of regions between two images is determined when test-statistics such as sum-of-squares, partial derivatives or absolute error/difference is within a predetermined threshold

**Claims**

1. An imaging element for providing image data, the imaging element comprising:

   - an imaging device configured to output image data,
   - a source of monochromatic radiation,
   - a movement sensor configured to output a movement signal, and
   - a processing device configured to receive the image data and the movement signal and output movement corrected image data.

2. An imaging element according to claim 1, wherein the imaging device has an imaging plane wherein the movement sensor is configured to output a movement signal correlated to movement of the imaging plane.

3. An imaging element according to claim 1 or 2, comprising a handheld unit comprising the imaging device and the movement sensor.

4. An imaging element according to any of the preceding claims, comprising a triggering element configured to activate the imaging device and the movement sensor.

5. An imaging element according to any of the preceding claims, wherein the movement sensor is configured to determine a distance between the imaging element and the imaged element and wherein the processing element is configured to include the distance in the determination of the corrected image data.

6. An imaging element according to any of the preceding claims, wherein the imaging device comprises a camera.

**7.** An imaging element according to any of the preceding elements, further comprising a display, the processing device being configured to control the display to display an image representing the corrected image data, the image illustrating colour relative movement between elements illustrated in different portions of the image.

**8.** An imaging element according to any of the preceding elements, wherein the source is a source of coherent radiation.

**9.** A method of obtaining image data of an element, the method comprising:

- launching monochromatic radiation on to the element,
- obtaining first image data of the element, using an imaging device, while determining movement of the imaging device,
- based on the determined movement and the first image data, generating movement corrected image data.

**10.** A method according to claim 9, wherein the imaging device has an imaging plane and wherein the step of determining movement comprises determining movement of the imaging plane vis-à-vis the imaged element.

**11.** A method according to any of claims 9 or 10, further comprising determining a distance from the imaging device to the imaged element, the generating step comprising generating the corrected image data also based on the distance determined.

**12.** A method according to any of claims 9-11, further comprising the step of displaying an image representing the corrected data, the image representing relative movement between different portions of the imaged element.

**13.** A method according to any of claims 9-12, wherein the launching step comprises illuminating the element with coherent radiation.

**Amended claims in accordance with Rule 137(2) EPC.**

**1.** An imaging element (10, 20) for providing image data, the imaging element comprising:

- an imaging device (58) configured to output image data,
- a source (57) of monochromatic radiation,
- a movement sensor (53, 59) configured to output a movement signal, and

- a processing device (55) configured to receive the image data and the movement signal and output movement corrected image data

**characterized in that** the movement sensor is configured to output a movement signal represending movement during the providing of the image.

**2.** An imaging element according to claim 1, wherein the imaging device has an imaging plane (201) wherein the movement sensor is configured to output a movement signal correlated to movement of the imaging plane.

**3.** An imaging element according to claim 1 or 2, comprising a handheld unit (50) comprising the imaging device and the movement sensor.

**4.** An imaging element according to any of the preceding claims, comprising a triggering element (56) configured to activate the imaging device and the movement sensor.

**5.** An imaging element according to any of the preceding claims, wherein the movement sensor (59) is configured to determine a distance between the imaging element and the imaged element and wherein the processing element is configured to include the distance in the determination of the corrected image data.

**6.** An imaging element according to any of the preceding claims, wherein the imaging device comprises a camera.

**7.** An imaging element according to any of the preceding elements, further comprising a display (40), the processing device being configured to control the display to display an image representing the corrected image data, the image illustrating colour relative movement between elements illustrated in different portions of the image.

**8.** An imaging element according to any of the preceding elements, wherein the source is a source of coherent radiation.

**9.** A method of obtaining image data of an element, the method comprising:

- launching monochromatic radiation on to the element,
- obtaining first image data of the element, using an imaging device, while determining movement of the imaging device,
- based on the determined movement and the first image data, generating movement corrected image data,

**characterized in that** the step of determining movement comprises determining movement during the obtaining of the first image data.

10. A method according to claim 9, wherein the imaging device has an imaging plane and wherein the step of determining movement comprises determining movement of the imaging plane vis-à-vis the imaged element.

11. A method according to any of claims 9 or 10, further comprising determining a distance from the imaging device to the imaged element, the generating step comprising generating the corrected image data also based on the distance determined.

12. A method according to any of claims 9-11, further comprising the step of displaying an image representing the corrected data, the image representing relative movement between different portions of the imaged element.

13. A method according to any of claims 9-12, wherein the launching step comprises illuminating the element with coherent radiation.

201    202    10                          242

|0                              0    241

20                                    22

30                              24

40

Figure 1

20

50              50

55    40    53        57    58    59

54                  54

56

Figure 2

Europäisches
Patentamt

European
Patent Office

Office européen
des brevets

## EUROPEAN SEARCH REPORT

Application Number

EP 18 19 6325

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | WO 2017/062759 A1 (VASOPTIC MEDICAL INC [US]) 13 April 2017 (2017-04-13) <br> * claims 1, 26, 43 * <br> * figure 2C * <br> * page 15, paragraph 1 - paragraph 2 * <br> * page 28, paragraph 2 * | 1-13 | INV. <br> A61B5/00 <br> A61B5/02 <br> H04N5/232 <br> G01S17/02 |
| A | US 2014/276099 A1 (KIRENKO IHOR OLEHOVYCH [NL] ET AL) 18 September 2014 (2014-09-18) <br> * claim 1 * <br> * figure 1 * <br> * paragraph [0056] * | 1-13 | |
| A | US 2017/354392 A1 (FENGLER JOHN J P [CA] ET AL) 14 December 2017 (2017-12-14) <br> * claim 1 * <br> * figure 1B * <br> * paragraph [0085] * <br> * paragraph [0128] * | 1-13 | |
| | | | TECHNICAL FIELDS SEARCHED (IPC) <br><br> A61B <br> H04N <br> G01S |

The present search report has been drawn up for all claims

| Place of search | Date of completion of the search | Examiner |
|---|---|---|
| The Hague | 28 February 2019 | Benzeroual, Karim |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.............................................................................

& : member of the same patent family, corresponding document

EPO FORM 1503 03.82 (P04C01)

**EP 3 626 160 A1**

## ANNEX TO THE EUROPEAN SEARCH REPORT
## ON EUROPEAN PATENT APPLICATION NO.

EP 18 19 6325

This annex lists the patent family members relating to the patent documents cited in the above-mentioned European search report.
The members are as contained in the European Patent Office EDP file on
The European Patent Office is in no way liable for these particulars which are merely given for the purpose of information.

28-02-2019

| Patent document cited in search report | Publication date | Patent family member(s) | Publication date |
|---|---|---|---|
| WO 2017062759 A1 | 13-04-2017 | AU 2016335761 A1 | 10-05-2018 |
| | | CA 3000530 A1 | 13-04-2017 |
| | | CN 108430306 A | 21-08-2018 |
| | | EP 3359016 A1 | 15-08-2018 |
| | | US 2018296103 A1 | 18-10-2018 |
| | | WO 2017062759 A1 | 13-04-2017 |
| US 2014276099 A1 | 18-09-2014 | BR 112015022109 A2 | 18-07-2017 |
| | | CN 105188520 A | 23-12-2015 |
| | | EP 2967378 A1 | 20-01-2016 |
| | | JP 2016513521 A | 16-05-2016 |
| | | RU 2015143896 A | 19-04-2017 |
| | | US 2014276099 A1 | 18-09-2014 |
| | | WO 2014141084 A1 | 18-09-2014 |
| US 2017354392 A1 | 14-12-2017 | CA 3027592 A1 | 21-12-2017 |
| | | US 2017354392 A1 | 14-12-2017 |
| | | WO 2017214730 A1 | 21-12-2017 |

EPO FORM P0459

For more details about this annex : see Official Journal of the European Patent Office, No. 12/82

**EP 3 626 160 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2017062759 A
- US 2017017858 A
- CN 107307848
- CN 204765619 U
- WO 2014009859 A
- WO 2011117779 A
- RU 2573053
- US 2016270672 A
- WO 2010017508 A
- CN 102357033

**Non-patent literature cited in the description**

- **LERTSAKDADET B et al.** Correcting for motion artifact in handheld laser speckle images. *Journal of Biomedical Optics,* March 2018, vol. 23, 036006
- Development of a handheld blood flow measurement system using laser speckle flowgraphy. **MIN-CHUL LEE et al.** Optical Society of Japan. Springer, April 2015, vol. 22, 308-314
- **FARRARO R et al.** Handheld, point-of-care laser speckle imaging. *Journal of Biomedical Optics,* September 2016, vol. 21, 094001
- **KUMAR MAYANK et al.** PulseCam: High-resolution blood perfusion imaging using a camera and a pulse oximeter. *38th Annual International Conference of the IEEE Engineering in Medicine and Biology Society (EMBC),* 16 August 2016, 3904-3909

19